# EUROPEAN PATENT APPLICATION

(11) **EP 4 257 689 A1**
(43) Date of publication of application: **11.10.2023**
(21) Application number: 21967447.0
(22) Date of filing: 29.12.2021
(51) Int. Cl.: C12N 15/63, C12N 9/90, C12N 15/70, C12P 19/02

(54) **NOVEL PROMOTER VARIANT FOR CONSTITUTIVE EXPRESSION AND USE THEREOF**

(71) Applicant: Daesang Corporation, Seoul 03130 (KR)
(72) Inventor: The designation of the inventor has not yet been filed
(74) Representative: Kehl, Ascherl, Liebhoff & Ettmayr Patentanwälte Partnerschaft mbB
(86) International application number: PCT/KR2021/020191
(87) International publication number: WO 2023/128004

(57) **Abstract**

The present invention provides a novel promoter variant in which some nucleotides of the glyceraldehyde-3-phosphate dehydrogenase (gapA) gene promoter of *Escherichia coli* are deleted. The novel promoter variant according to the present invention can constitutively express a target protein at a high level, particularly an enzyme, in *Escherichia coli.* Therefore, a target protein, particularly an enzyme, can be economically mass-produced using a recombinant strain transformed with an expression vector containing the novel promoter variant according to the present invention. For example, allulose epimerase can be economically mass-produced or allulose can be economically mass-produced from fructose using a recombinant strain transformed with an expression vector containing the novel promoter variant according to the present invention.

## Description

### [Technical Field]

The present invention relates to novel promoter variants and the like, and more particularly, to novel promoter variants capable of consistently high-expressing a target protein and various uses thereof.

### [Background Art]

With advances in molecular biology, several mechanisms for regulating gene expression have been identified. Gene expression refers to a series of processes of synthesizing proteins according to codes input into genes through transcription and translation occurring in cells. In particular, it is known that the transcription process is an early step in gene expression, it is initiated by RNA polymerase binding to the promoter sequence located upstream of the gene with the help of several cofactors, and transcription factor (TF), which is one of such cofactors, binds directly to the promoter sequence. In particular, regulation of gene expression in prokaryotes mainly occurs at the transcriptional level, so researchers are continuously discovering new transcription factors and promoters.

In order to industrially produce foreign proteins such as enzymes, a transformant prepared by transforming a prokaryotic organism such as *Escherichia coli* with a pET-type expression vector containing a foreign protein gene is used as an expression system. A prokaryotic expression system transformed with a pET-type expression vector has a disadvantage in that in general, expensive expression inducers such as isopropyl-β-D-thiogalactopyranoside (IPTG) are required, and the concentration of the inducer or equipment, expression induction time, and the like must be precisely controlled.

Meanwhile, in order to mass-produce psicose epimerase (or allulose epimerase) having an activity that converts fructose into allulose (or psicose), an expression system using a strain of the genus *Corynebacterium,* generally recognized as safe (GRAS) strain, as a host cell has been proposed. Regarding the psicose epimerase (or allulose epimerase) expression system based on the *Corynebacterium* genus strain, Korean Patent Registration No. 10-1656063 discloses a nucleotide sequence encoding a psicose epimerase and a gene expression cassette including a regulatory sequence operably linked upstream thereof and regulating the expression of the psicose epimerase in a strain of the genus *Corynebacterium,* and the regulatory sequence is composed of a transcriptional promoter, a first ribosome binding region (RBS) sequence, a first space sequence, a linker sequence, and a second RBS sequence. In addition, Korean Patent Registration No. 10-1695830 discloses a nucleic acid sequence encoding the psicose epimerase and a gene expression cassette including a regulatory sequence operably linked upstream thereof and regulating the expression of the psicose epimerase in a strain of the genus *Corynebacterium,* and the regulatory sequence includes an *E.coli-*derived transcription promoter. However, the *Corynebacterium-based* psicose epimerase (or allulose epimerase) expression system is not suitable for a large-scale enzyme expression system because the expression level of promoters that can be used is low and the selection range is small.

Therefore, for mass production of psicose epimerase (or allulose epimerase) or mass production of allulose from fructose using psicose epimerase (or allulose epimerase), there is a need to develop a constitutive expression promoter capable of stable high-level expression of foreign proteins without growth inhibition under general culture conditions of *E. coli* host cells and a constitutive expression system including the same.

### [Disclosure]

### [Technical Problem]

The present invention has been derived under the conventional technical background, and an object of the present invention is to provide a novel promoter variant capable of consistently expressing a target protein at a high level. Further, an object of the present invention is to provide various uses of the novel promoter variants.

### [Technical Solution]

The inventors of the present invention operably link the gapA gene promoter, which is a promoter for expressing the glyceraldehyde-3-phosphate dehydrogenase (gapA) gene of *Escherichia coli,* with a polynucleotide encoding allulose epimerase, thereby constructing a recombinant expression vector and introduce the recombinant expression vector into *E. coli* for transformation. At this time, random variations occurred in the gapA promoter or allulose epimerase gene sequence, so the inventors of the present invention secured recombinant *E. coli* transformed with various recombinant expression vectors different from the introduced recombinant expression vector. As a result of comparing the allulose epimerase expression activity of recombinant *E. coli* transformed with a recombinant expression vector in which there was no mutation in the allulose epimerase gene sequence and only in the gapA gene promoter among the recombinant expression vectors obtained through the random variation, it was confirmed that a promoter variant in which adenine (A), the 130th nucleotide of the nucleotide sequence of the gapA gene promoter, was deleted could constitutively and highly express allulose epimerase, thereby completing the present invention.

In order to achieve the above object, one example of the present invention provides a promoter variant consisting of the nucleotide sequence represented by SEQ ID NO: 9. Further, one example of the present invention provides a recombinant vector including a promoter variant consisting of the nucleotide sequence represented by SEQ ID NO: 9. Further, one example of the present invention provides an expression vector including a polynucleotide encoding a target protein and the promoter variant operably linked thereto and consisting of the nucleotide sequence represented by SEQ ID NO: 9. Further, one example of the present invention provides a recombinant strain transformed with the expression vector. Further, one example of the present invention provides a method for producing a target protein using the recombinant strain. Further, one preferred example of the present invention provides a method for producing an enzyme conversion reaction product from a substrate, the method including the step of adding the recombinant strain to a substrate-containing solution and proceeding with the enzyme conversion reaction.

### [Advantageous Effects]

The novel promoter variant according to the present invention can constitutively express a target protein, particularly an enzyme, in *Escherichia coli.* Therefore, a target protein, particularly an enzyme, can be economically mass-produced using a recombinant strain transformed with an expression vector containing the novel promoter variant according to the present invention. For example, allulose epimerase can be economically mass-produced or allulose can be economically mass-produced from fructose using a recombinant strain transformed with an expression vector containing the novel promoter variant according to the present invention.

### [Description of Drawings]

FIG. 1 shows a cleavage map of the recombinant expression vector pPgapAm1-FpDPE [W29K/A77S/G216S/M234I] prepared in Example of the present invention.
FIG. 2 shows a cleavage map of the recombinant expression vector pPgapAm2-FpDPE [W29K/A77S/G216S/M234I] prepared in Example of the present invention.
FIG. 3 shows a cleavage map of the recombinant expression vector pPblma-FpDPE [W29K/A77S/G216S/M234I] prepared in Example of the present invention.
FIG. 4 shows the conversion rate according to the reaction time when the conversion reaction of fructose to allulose is carried out using the recombinant *E. coli* prepared in Example of the present invention.

### [Detailed Description of Embodiment]

Hereinafter, the present invention is described in detail.

As used herein, the term 'promoter' refers to a minimum nucleic acid sequence that is operably linked to a target nucleotide sequence to be transcribed and regulates transcription of the target nucleotide sequence. Further, the promoter may include promoter constructs sufficient to allow expression of a regulatable promoter-dependent gene induced by a cell type-specific or external signal or agent, and these constructs may be located in the 5' or 3' portion of the gene. The promoter includes both conserved promoters and inducible promoters. Promoter sequences can be derived from prokaryotes, eukaryotes, and viruses. A promoter in prokaryotes is usually defined as a binding site immediately adjacent to a transcription start site where RNA polymerase binds.

As used herein, the term 'a promoter variant' is defined as a promoter having a different or improved target protein expression activity from that of the basic promoter by deletion, addition or substitution of some nucleotides in the nucleic acid sequence of the basic promoter.

As used herein, the term 'homologous' indicates identity with a nucleic acid sequence of a wild type or a variant having the same activity, and comparison of homology may be calculated as a percentage (%) of homology between two or more sequences with the naked eye or using a comparison program that is easy to purchase.

As used herein, the term 'target protein' refers to a protein that cannot normally exist in a transformed strain (or host cell) expressing the protein as an exogenous protein.

As used herein, the term 'polynucleotide' refers to any polyribonucleotide (RNA) or polydeoxyribonucleotide (DNA) that is non-modified or modified. The polynucleotide includes single- or double-stranded DNA, DNA as a mixture of single- and double-stranded regions, single- and double-stranded RNA, RNA as a mixture of single- and double-stranded regions, or hybrid molecules thereof, but it is not limited thereto.

As used herein, the term 'operably linked' is defined as a state in which a promoter sequence and a nucleotide sequence encoding a target protein are functionally linked so that the promoter can control the expression of the target protein. For example, when a promoter is capable of controlling the expression of a coding sequence (i.e., when the coding sequence is under the transcriptional control of a promoter), the promoter is operably linked with the coding sequence, or the ribosome binding site is positioned so as to facilitate translation, meaning that the ribosome binding site is operably linked with the coding sequence. Coding sequences may be operatively linked to regulatory sequences in either the sense or antisense orientation.

As used herein, the term 'recombinant vector' is defined as a recombinant DNA prepared by excising a promoter variant or target gene using a restriction enzyme and inserting it into a vector.

As used herein, the term 'cloning vector' is defined as a material capable of transporting a DNA fragment into a host cell and reproducing it. The cloning vector may include a polyadenylation signal, a transcription termination sequence, and multiple cloning sites. The multiple cloning site includes at least one endonuclease restriction enzyme restriction site. In addition, the cloning vector may further include a promoter. Further, a polynucleotide encoding a target protein in a cloning vector may be located upstream of a polyadenylation signal and a transcription termination sequence.

As used herein, the term 'expression vector' is defined as a DNA sequence required for transcription and translation of cloned DNA in an appropriate host, and specifically, it refers to a genetic construct including essential regulatory elements operably linked to an insert such that the insert is expressed when present in a cell of a subject. Expression vectors may be prepared and purified using standard recombinant DNA techniques. The type of the expression vector is not particularly limited as long as it functions to express a desired gene and produce a desired protein in various host cells such as prokaryotic and eukaryotic cells. An expression vector includes at least a promoter, a start codon, a gene encoding a desired protein, and a stop codon terminator. In addition, the expression vector may also contain, as appropriate, DNA encoding a signal peptide, additional expression regulatory sequences, untranslated regions on the 5' and 3' sides of a desired gene, a selection marker region, a replicable unit, or the like. The selection marker region may be an antibiotic selection marker gene for selecting a desired vector.

As used herein, the term 'recombinant strain' refers to a cell transformed by introducing a polynucleotide encoding one or more target proteins or an expression vector having the same into a host cell. Methods for preparing transformants by introducing the expression vector into host cells include transient transfection, microinjection, transduction, cell fusion, calcium phosphate precipitation, liposome-mediated transfection, diethylaminoethyl (DEAE) dextran-mediated transfection, polybrene-mediated transfection, electroporation, electroinjection, a chemical treatment method using polyethylene glycol (PEG), a method using a gene gun, a heat shock method, and the like, but are not limited thereto.

As used herein, the term 'substrate' refers to any substance or compound that is converted or is to be converted into another compound by the action of an enzyme. The term encompasses single compounds as well as combinations of compounds and their derivatives, such as solvents, mixtures and other materials containing at least one substrate.

One aspect of the present invention relates to a novel promoter variant capable of constitutively expressing a target protein at a high level. A novel promoter variant according to an embodiment of the present invention is composed of the nucleotide sequence represented by SEQ ID NO: 9. The novel promoter variant according to an embodiment of the present invention is a mutation of the promoter of the gene gapA (glyceraldehyde-3-phosphate dehydrogenase) derived from *Escherichia coli* composed of the nucleotide sequence represented by SEQ ID NO: 1, in particular, adenine, which is the 130th nucleotide of the nucleotide sequence represented by SEQ ID NO: 1, is deleted. Adenine, which is the 130th nucleotide of the nucleotide sequence represented by SEQ ID NO: 1, corresponds to a ribosome-binding site (RBS). An expression system including a novel promoter variant according to an embodiment of the present invention may constitutively express a target protein at high levels in *E. coli.* Therefore, the novel promoter variant according to an embodiment of the present invention may be used as a promoter for constitutive expression. The novel promoter variant according to an embodiment of the present invention consists of the nucleotide sequence represented by SEQ ID NO: 9, but the equivalent range of the novel promoter variant according to an embodiment of the present invention is not necessarily limited thereto. For example, the equivalent range of novel promoter variants according to an embodiment of the present invention includes a promoter in which some nucleotides of the nucleotide sequence represented by SEQ ID NO: 9 are substituted, inserted, and/or deleted within the range of maintaining the function of constitutively high expression of the target protein. Further, the equivalent range of the novel promoter variant according to an embodiment of the present invention includes a sequence having substantial identity to the nucleotide sequence represented by SEQ ID NO: 9. The above "substantial identity" means that the nucleotide sequence represented by SEQ ID NO: 9 and any other sequence are aligned so as to correspond as much as possible, the sequences are analyzed, and the any other sequence has a sequence homology of 70% or more, 90% or more or 98% or more of the nucleotide sequence represented by SEQ ID NO: 9. A person skilled in the art can easily understand that a polynucleotide having the same or similar activity within the scope of substantial homology may be prepared by substituting, adding, or deleting one or more nucleotides in the nucleotide sequence of the novel promoter variant using genetic recombination technology known in the art, etc. Comparison of such homology may be performed by calculating homology between two or more sequences as a percentage (%) using a commercially available computer program. Therefore, the equivalent range of the novel promoter variant according to an embodiment of the present invention may include a nucleotide sequence having 70% or more, 80% or more, 90% or more, 95% or more, or 99% or more homology with the nucleotide sequence represented by SEQ ID NO: 9 within the range in which the function of consistently high expression of the target protein is maintained.

Another aspect of the present invention relates to various uses of the novel promoter variant according to an embodiment of the present invention. The use of the novel promoter variant according to an embodiment of the present invention includes a recombinant vector, an expression vector, a recombinant strain, a method for producing a target protein, and a method for demonstrating the function of a target protein using a recombinant strain, but it is not necessarily limited thereto.

A recombinant vector according to an embodiment of the present invention includes a promoter variant consisting of the nucleotide sequence represented by SEQ ID NO: 9.

The recombinant vector may be a cloning vector. The cloning vector may include an origin of replication, a multiple cloning site (MCS) for cloning a target protein gene, a transcription termination sequence, and a selection marker. The selection marker is for selecting cells transformed with the vector, and markers conferring selectable phenotypes such as drug resistance, auxotrophy, resistance to cytotoxic agents, or surface protein expression may be used. Since only cells expressing a selection marker survive in an environment treated with a selective agent, transformed cells can be selected. For example, the selection marker may be a drug resistance gene such as a kanamycin antibiotic resistance gene or an ampicillin antibiotic resistance gene.

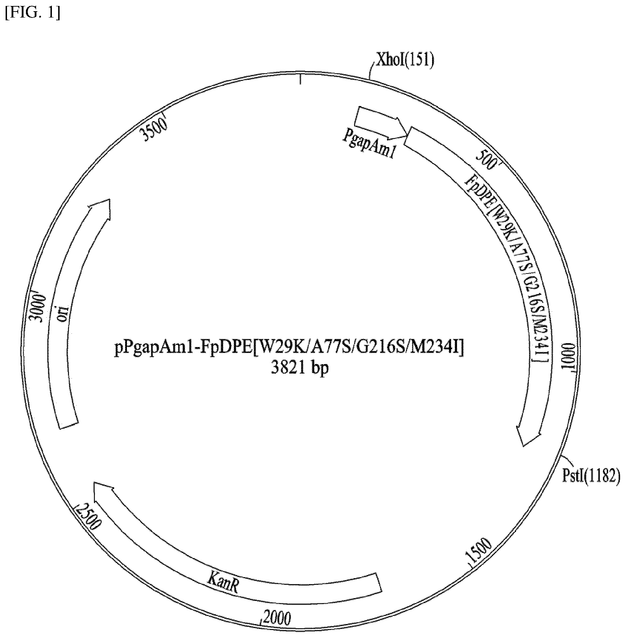

Further, the recombinant vector may be an expression vector. The expression vector includes a polynucleotide encoding a target protein and the promoter variant operably linked thereto and consisting of the nucleotide sequence represented by SEQ ID NO: 9. The promoter variant is preferably located upstream of the polynucleotide encoding the target protein. The type of target protein expressed through the expression vector according to an embodiment of the present invention is not significantly limited, but may be selected from, for example, proteins involved in the biosynthesis or metabolism of carbohydrates, proteins involved in the biosynthesis or metabolism of lipids and fatty acids, proteins involved in the biosynthesis or metabolism of proteins and peptides, or the like. Further, the target protein is preferably an enzyme in consideration of the expression control activity of the promoter variant. The type of enzyme is not particularly limited, but may be selected from D-allulose 3-epimerase, D-tagatose 3-epimerase, (1→4)-alpha-D-glucan 1-alpha-D-glucosylmutase, 4-alpha-D-{(1→4)-alpha-D-glucano}trehalose trehalohydrolase, L-rhamnose isomerase, fructose-6-phosphate-3-epimerase, and the like, and is preferably selected from D-allulose 3-epimerase, (1→4)-alpha-D-glucan 1-alpha-D-glucosylmutase, 4-alpha-D-{(1→4)-alpha-D-glucano}trehalose trehalohydrolase, and the like. Although not specifically described in the examples of the present invention, the novel promoter variant of the present invention are prepared by expressing (1→4)-alpha-D-glucan 1-alpha-D-glucosylmutase, 4-alpha-D-{(1→4)-alpha-D-glucano}trehalose trehalohydrolase, and the like at a level of about 1.4 to 1.5 times higher, compared to the promoter of the gene gapA (glyceraldehyde-3-phosphate dehydrogenase) derived from *Escherichia coli* composed of the nucleotide sequence represented by SEQ ID NO: 1, The type of the allulose epimerase is not particularly limited as long as it has an activity that converts fructose into allulose, and for example, it may consist of the amino acid sequence represented by SEQ ID NO: 3, the amino acid sequence represented by SEQ ID NO: 5 or the amino acid sequence represented by SEQ ID NO: 7. Further, the polynucleotide encoding the allulose epimerase may consist of the nucleotide sequence represented by SEQ ID NO: 4, the nucleotide sequence represented by SEQ ID NO: 6, or the nucleotide sequence represented by SEQ ID NO: 8. Further, the present invention includes information disclosed in Korean Patent Registration No. 10-1919713, Korean Patent Registration No. 10-2187354, Korean Patent Registration No. 10-1656063, Korean Patent Registration No. 10-1695830, Korean Patent Registration No. 10-2189458, Korean Patent Registration No. 10-1539097, Korean Patent Registration No. 10-1539096, Korean Patent Registration No. 10-1455759, Korean Patent Registration No. 10-1318422 and the like in relation to an allulose epimerase and a polynucleotide encoding the same. An expression vector according to a preferred embodiment of the present invention has the cleavage map of FIG. 1. The expression vector having the cleavage map shown in FIG. 1 has a structure in which pUC-derived replication origin (ori), a promoter variant consisting of the nucleotide sequence represented by SEQ ID NO: 9 (PgapAm1), a polynucleotide (FpDPE [W29K/A77S/G216S/M234I]) encoding an allulose epimerase consisting of the nucleotide sequence represented by SEQ ID NO: 8, and kanamycin resistance gene marker (KanR) and the like are sequentially connected.

A recombinant strain according to an embodiment of the present invention is prepared by transforming a host cell by introducing an expression cassette consisting of a polynucleotide encoding a target protein and a promoter variant operably linked thereto and composed of the nucleotide sequence represented by SEQ ID NO: 9 or an expression vector containing the expression cassette. In the present invention, the type of host cell that can be transformed with the expression vector is not particularly limited as long as the novel promoter variant according to an embodiment of the present invention can operate smoothly, and it is preferably a prokaryotic cell and more preferably *E. coli* in view of the expression control activity of the novel promoter variant, the efficiency of DNA introduction, the expression efficiency of the introduced DNA, and the like. Examples of *Escherichia coli* include BL21, JM109, K-12, LE392, RR1, DH5α, W3110, or the like, but are not limited thereto. The recombinant strain according to an example of the present invention is preferably recombinant *Escherichia coli* DS00004 (accession number: KCCM13092P; accession date: December 14, 2021) deposited with the Korean Culture Center of Microorganisms, an international depository organization. The recombinant *Escherichia coli* DS00004 (accession number: KCCM13092P; accession date: December 14, 2021) is prepared by transforming the host cell *E. coli* DH5α by introducing a recombinant expression vector pPgapAm1-FpDPE [W29K/A77S/G216S/M234I] having the cleavage map of FIG. 1.

A method for producing a target protein according to an embodiment of the present invention includes culturing the above-described recombinant strain to express the target protein; and isolating the target protein from the culture medium of the recombinant strain or the cells of the recombinant strain. Depending on the type, the target protein may be present in the cells of the recombinant strain or secreted out of the cells of the recombinant strain after expression. For example, when the target protein is allulose epimerase, the allulose epimerase produced by the recombinant strain is present in the cells of the recombinant strain. A method for producing an allulose epimerase according to a preferred embodiment of the present invention includes culturing a recombinant strain transformed by introducing an expression cassette consisting of a polynucleotide encoding an allulose epimerase and a promoter variant operably linked thereto and composed of the nucleotide sequence represented by SEQ ID NO: 9 or an expression vector containing the expression cassette to express allulose epimerase; and isolating the allulose epimerase from the lysate of the recombinant strain in which the allulose epimerase is expressed. The novel promoter variant according to an embodiment of the present invention is a constitutive expression vector, and thus expression may be induced without using isopropyl-1-thio-β-D-galactopyranoside (IPTG), or the like, which is a protein expression inducing factor. In the present invention, allulose epimerase may be recovered from the lysate of the recombinant strain. Cells used for protein expression may be disrupted by various material or chemical means, such as freeze-thaw cycles, sonication, mechanical disruption, or cytolytic agents and may be isolated or purified by conventional biochemical separation techniques (Sambrook et al., Molecular Cloning: A laboratory Manual, 2nd Ed., Cold Spring Harbor Laboratory Press, 1989; Deuscher, M., Guide to Protein Purification Methods Enzymology, Vol. 182. Academic Press. Inc., San Diego, CA, 1990). For example, methods for isolating or purifying proteins expressed by host cells include electrophoresis, centrifugation, gel filtration, precipitation, dialysis, chromatography (ion exchange chromatography, affinity chromatography, immunosorbent affinity chromatography, reverse phase high-performance liquid chromatography (HPLC), gel permeation HPLC), isoelectric focusing and various variations or combinations thereof methods, but are not limited thereto. Meanwhile, in the present invention, the step of isolating allulose epimerase from the lysate of the recombinant strain may be preferably performed by affinity chromatography using a peptide tag. As the peptide tag, a variety of well-known tags such as an HA tag, FLAG tag, His tag, biotin carboxyl carrier protein (BCCP), c-myc tag, V5 tag, glutathione-S-transferase (GST) or maltose binding protein (MBP) may be used, and a double His tag is preferable. His-tagged proteins are specifically trapped on a column of nickel-nitrilotriacetic acid (Ni-NTA) resin and may be eluted with ethylenediaminetetraacetic acid (EDTA) or imidazole.

A recombinant strain according to an embodiment of the present invention may be used to indirectly exert the function of a target protein in addition to being used to produce a target protein. For example, when the target protein is an enzyme, the recombinant strain according to an embodiment of the present invention may be used to prepare a product by an enzyme conversion reaction from a substrate. Specifically, when the target protein is allulose epimerase, the present invention provides a method for preparing allulose from fructose, including adding and reacting a recombinant strain to a fructose-containing solution. The recombinant strain is a host cell transformed by introduction of an expression cassette consisting of a polynucleotide encoding allulose epimerase and a promoter variant operably linked thereto and composed of the nucleotide sequence represented by SEQ ID NO: 9, or an expression vector containing the expression cassette and is preferably recombinant *Escherichia coli* DS00004 (accession number: KCCM13092P; accession date: December 14, 2021). Further, the fructose-containing solution may further include metal ions such as Ca²⁺ and Mn²⁺ to promote the activity of allulose epimerase. Further, the reaction temperature in the method for producing allulose from fructose is in the range of 60°C to 70°C, preferably 60°C to 67°C, and more preferably 60°C to 65°C, considering the smooth enzyme expression of the recombinant strain, the stability and maximum activity of the enzyme, and the reaction pH is in the range of 6.5 to 8, preferably 6.5 to 7.5, and more preferably 6.5 to 7. Further, the concentration of fructose in the method for producing allulose from fructose is not particularly limited, but considering productivity or economy, it is preferably 1 to 75% (w/w), and more preferably 4 to 35% (w/w) based on the total reactants.

Hereinafter, the present invention is described in more detail through Examples. However, the following Examples are only for clearly illustrating the technical features of the present invention, but do not limit the protection scope of the present invention.

### Example 1: Securing a Promoter for Enzyme Gene Expression

### 1.1 Securing gapA (glyceraldehyde-3-phosphate dehydrogenase) gene promoter

The gapA (glyceraldehyde-3-phosphate dehydrogenase) gene promoter is a promoter for expressing the gapA (glyceraldehyde-3-phosphate dehydrogenase) gene of *Escherichia coli,* and it consists of the nucleotide sequence represented by SEQ ID NO: 1 and is known as a promoter that induces strong constitutive expression in *Escherichia coli* [See. Thouvenot et al (2004) The strong efficiency of the Escherichia coli gapA P1 promoter depends on a complex combination of functional determinants, Biochemical Journal 383:371-382. DOI: 10.1042/BJ20040792].

In order to secure a polynucleotide fragment corresponding to the promoter region of the gapA (glyceraldehyde-3-phosphate dehydrogenase) gene from *Escherichia coli,* polymerase chain reaction (PCR) was performed using the genomic DNA of *Escherichia coli* MG1655 as a template and the primer set shown in Table 1 below. The obtained amplification product was cloned into a pGEM-Teasy vector (Promega Co., USA), and the nucleotide sequence was analyzed. The result of analysis confirmed the polynucleotide fragment having a length of 137 bp and consisting of the nucleotide sequence represented by SEQ ID NO: 1.

**[Table 1]**

| Primer Name | Primer Description | Primer Sequence (5'→3') |
|---|---|---|
| PgapA-F | gapA promoter forward primer | AGGCGAGTCAGTCGCGTAAT |
| PgapA-R | gapA promoter reverse primer | ATATTCCTCCAGCTATTTGTTAG |

### 1.2 Securing BLMA gene promoter

The BLMA gene promoter is a promoter for expressing the maltogenic amylase gene of *Bacillus licheniformis,* and it consists of the nucleotide sequence represented by SEQ ID NO: 2 and is known as a promoter that induces stable high expression of foreign genes in *Escherichia coli* [See. TAE-JIP KIM et al (1999) Modes of Action of Acarbose Hydrolysis and Transglycosylation Catalyzed by a Thermostable Maltogenic Amylase, the Gene for Which Was Cloned from a Thermus Strain].

In order to secure a polynucleotide fragment corresponding to the promoter region of the maltogenic amylase gene from *Bacillus licheniformis,* a polymerase chain reaction (PCR) was performed using the genomic DNA of *Bacillus licheniformis* ATCC 14580 as a template and the primer set shown in Table 1 below. The obtained amplification product was cloned into a pGEM-Teasy vector (Promega Co., USA), and the nucleotide sequence was analyzed. The result of analysis confirmed the polynucleotide fragment having a length of 115 bp and consisting of the nucleotide sequence represented by SEQ ID NO: 2.

**[Table 2]**

| Primer Name | Primer Description | Primer Sequence (5'→3') |
|---|---|---|
| Pblma-F | BLMA promoter forward primer | GGTGTCTCATTCTGTTACCG |
| Pblma-R | BLMA promoter reverse primer | GTTTCCCCCTTTTGGTTGTC |

### Example 2: Securing allulose epimerase variant cloning vector

The applicant of the present invention disclosed a wild-type D-allulose epimerase derived from *Flavonifractor plautii* and a polynucleotide encoding the same through Korean Patent Registration No. 10-14739180. The wild-type D-allulose epimerase consists of the amino acid sequence represented by SEQ ID NO: 3, and the polynucleotide encoding it consists of the nucleotide sequence represented by SEQ ID NO: 4.

Further, the applicant of the present invention disclosed a D-allulose epimerase variant W29K/G216S/M234I with improved conversion rate of fructose to allulose and thermal stability and a polynucleotide encoding the same through Korean Patent Publication No. 10-2021-0132405. In the D-allulose epimerase variant W29K/G216S/M234I, tryptophan (Trp) is substituted with lysine (Lys) at 29th position, glycine (Gly) is substituted with serine (Ser) at the 216th position, and at the same time, methionine (Met) is substituted with isoleucine (Ile) at the 234th position in the amino acid sequence of wild-type D-allulose epimerase derived from *Flavonifractor plautii* and the D-allulose epimerase variant W29K/G216S/M234I consists of the amino acid sequence represented by SEQ ID NO: 5, and the polynucleotide encoding it consists of the nucleotide sequence represented by SEQ ID NO: 6.

Further, the applicant of the present invention derived the D-allulose epimerase variant W29K/A77S/G216S/M234I, which has excellent thermal stability under high temperature conditions, and filed a patent application on December 14, 2021 (Korean Patent Application No. 10-2021-0178690, unpublished). In the D-allulose epimerase variant W29K/A77S/G216S/M234I, tryptophan (Trp) is substituted with lysine (Lys) at 29th position, alanine (Als) is substituted with serine (Ser) at the 77th position, glycine (Gly) is substituted with serine (Ser) at the 216th position, and at the same time, methionine (Met) is substituted with isoleucine (Ile) at the 234th position in the amino acid sequence of wild-type D-allulose epimerase derived from *Flavonifractor plautii* and the D-allulose epimerase variant W29K/A77S/G216S/M234I consists of the amino acid sequence represented by SEQ ID NO: 7, and the polynucleotide encoding it consists of the nucleotide sequence represented by SEQ ID NO: 8.

Polynucleotide fragments encoding the amino acid sequences of the D-allulose epimerase variants W29K/A77S/G216S/M234I were prepared using the overlap extension polymerase chain reaction method based on the polynucleotide (SEQ ID NO: 6) of the D-allulose epimerase variant W29K/G216S/M234I. Specifically, 1 pM of oligonucleotide primers (A77S forward primer, A77S reverse primer) of Table 3 below and 100 ng of polynucleotide (SEQ ID NO: 6) of D-allulose epimerase variant W29K/G216S/M234I used as a template (template) were mixed to the reaction solution to which 100 µM of deoxynucleotide triphosphate (dATP, dCTP, dGTP, dTTP), and PCR reaction was performed at 25 to 30 cycles in the presence of 1 unit of pfu-X DNA polymerase mixture (Bioneer) using a thermocycler (TP600, TAKARA BIO Inc., JAPAN). Variant fragments are amplified through primer combinations, and then the amplified fragment was used as a template, and oligonucleotide primers (NdeI forward primer, XhoI reverse primer) introduced with the NdeI and XhoI restriction enzyme recognition site sequences in Table 3 below were used to finally construct a polynucleotide fragment (SEQ ID NO: 8) encoding the amino acid sequence of the D-allulose epimerase variant W29K/A77S/G216S/M234I through overlap extension PCR. Thereafter, the prepared polynucleotide fragment was inserted into the same restriction enzyme site of the pET28a vector (Novagen) using restriction enzymes NdeI and XhoI to secure a cloning vector.

**[Table 3]**

| Primer Description | Primer Sequence (5'→3') |
|---|---|
| A77S forward primer | AATACGACCTGAGCAGCGACGATCCGGCGGTG |
| A77S reverse primer | GATCGTCGCTGCTCAGGTCGTATTTGGCCTCCA |
| NdeI forward primer | GCATGCCATATGAACCCGATTGGAATGCA |
| XhoI reverse primer | GCATGCCTCGAGCGCGGTCAGCTCCTTGAGGA |

### Example 3: Preparation of Linked Fragment of Promoter and Allulose Epimerase Variant Gene

### 3.1 Preparation of DNA fragment in which apA promoter and allulose epimerase variant gene are linked

To amplify the gapA promoter, PCR was performed using the pGEM-Teasy vector cloned with the gapA promoter obtained in Example 1 as a template and using the primer sets (XhoI-PgapA, PgapA-FpDPE_R) shown in Table 4 below.

In addition, to amplify the gene of W29K/A77S/G216S/M234I, a D-allulose epimerase variant derived from *Flavonifractor plautii,* PCR was performed using pET28a vector (Novagen) cloned with the gene of W29K/A77S/G216S/M234I, a D-allulose epimerase variant as a template and using the primer sets (PgapA-FpDPE_F, PstI-FpDPE) shown in Table 4 below.

**[Table 4]**

| Primer Name | Primer Description | Primer Sequence (5'→3') |
|---|---|---|
| XhoI-PgapA | forward primer | TTTGCGCTCGAGAGGCGAGTCAGTCGC GTAAT |
| PgapA-FpDPE_R | reverse primer | TGCATTCCAATCGGGTTCATATATTCCTC CAGCTATTTGT |
| PgapA-FpDPE_F | forward primer | ACAAATAGCTGGAGGAATATATGAACCC GATTGGAATGCA |
| PstI-FpDPE | reverse primer | GCATGCTGCAGTTACGCGGTCAGCTCCT TGA |

The gapA promoter fragment thus amplified, and the D-allulose epimerase variant gene fragment may be linked into one fragment by the complementary sequences of the primers used for amplification. The two fragments as templates and primers (XhoI-PgapA, PstI-FpDPE) introduced with the XhoI and PstI restriction enzyme recognition site sequences in Table 4 were used to perform overlap extension PCR, thereby obtaining one amplified fragment. A DNA fragment in which the obtained gapA promoter and the gene of the allulose epimerase variant W29K/A77S/G216S/M234I were linked was named 'PgapA-FpDPE'.

### 3.2 Preparation of DNA fragment in which BLMA promoter and allulose epimerase variant gene are linked

To amplify the BLMA promoter, PCR was performed using the pGEM-Teasy vector cloned with the BLMA promoter obtained in Example 1 as a template and using the primer sets (XhoI-Pblma, Pblma-FpDPE_R) shown in Table 5 below.

In addition, to amplify the gene of W29K/A77S/G216S/M234I, a D-allulose epimerase variant derived from *Flavonifractor plautii,* PCR was performed using pET28a vector (Novagen) cloned with the gene of W29K/A77S/G216S/M234I, a D-allulose epimerase variant as a template and using the primer sets (Pblma-FpDPE_F, PstI-FpDPE) shown in Table 5 below.

**[Table 5]**

| Primer Name | Primer Description | Primer Sequence (5'→3') |
|---|---|---|
| XhoI-Pblma | forward primer | TTTGCGCTCGAGGGTGTCTCATTCTGTTA CCG |
| Pblma-FpDPE_R | reverse primer | TGCATTCCAATCGGGTTCATGTTTCCCCC TTTTGGTTGTC |
| Pblma-FpDPE_F | forward primer | GACAACCAAAAGGGGGAAACATGAACC CGATTGGAATGCA |
| PstI-FpDPE | reverse primer | GCATGCTGCAGTTACGCGGTCAGCTCCT TGA |

The BLMA promoter fragment thus amplified, and the D-allulose epimerase variant gene fragment may be linked into one fragment by the complementary sequences of the primers used for amplification. The two fragments as templates and primers (XhoI-Pblma, PstI-FpDPE) introduced with the XhoI and PstI restriction enzyme recognition site sequences in Table 5 were used to perform overlap extension PCR, thereby obtaining one amplified fragment. A DNA fragment in which the obtained BLMA promoter and the gene of the allulose epimerase variant W29K/A77S/G216S/M234I were linked was named 'Pblma-FpDPE.'

### Example 4: Preparation of D-allulose epimerase variant expression vector

### 4.1 Preparation of D-allulose epimerase variant expression vector having gapA promoter

A recombinant plasmid vector containing pUC-derived replication origin that can be replicated in *E. coli* through genetic manipulation from the pTrc99A vector (Pharmacia, US), a multiple cloning site (MCS) such as restriction enzymes XhoI site and PstI site, a transcription terminator, and a kanamycin antibiotic resistance gene was constructed. Then, the polynucleotide fragment PgapA-FpDPE prepared in Example 3 was digested with restriction enzymes XhoI and PstI, and then ligated with the recombinant plasmid vector having the same restriction enzyme site to prepare D-allulose epimerase variant expression vector pPgapA-FpDPE [W29K/A77S/G216S/M234I]. Thereafter, the D-allulose epimerase variant expression vector was transformed into *E. coli* DH5α by a heat shock method (See Sambrook and Russell: Molecular Cloning) to secure kanamycin-resistant colonies, six colonies were selected, then six recombinant expression vectors pPgapA-FpDPE [W29K/A77S/G216S/M234I] were recovered, and their nucleotide sequences were analyzed. As a result of sequence analysis of six recombinant expression vectors of pPgapA-FpDPE [W29K/A77S/G216S/M234I], random variations were confirmed in the gene sequence of the introduced gapA promoter or allulose epimerase variant W29K/A77S/G216S/M234I, respectively, and random variation contents are shown in Table 6 below.

**[Table 6]**

| Colony Classification | gapA Promoter Sequence Variant Content | W29K/A77S/G216S/M234I Gene Sequence Variation Content |
|---|---|---|
| Colony 1 | 130th nucleotide (A) 1 bp deletion/corresponding to ribosome-binding site (RBS) variation | Same |
| Colony 2 | Same | 1st nucleotide (A) 1 bp deletion/corresponding to loss of start codon (ATG) |
| Colony 3 | Same | 4th nucleotide (A) 1 bp deletion/corresponding to frameshift mutation |
| Colony 4 | Same | 10th nucleotide (A) 1 bp deletion/corresponding to frameshift mutation |
| Colony 5 | 132nd to 136th nucleotide (GAATA) 5 bp deletion/corresponding to RBS variation | Same |
| Colony 6 | Same | 168th nucleotide substitution variation (C→A)/corresponding to the acquisition of the stop codon (TGA) and ending transcription after the 55th alanine |

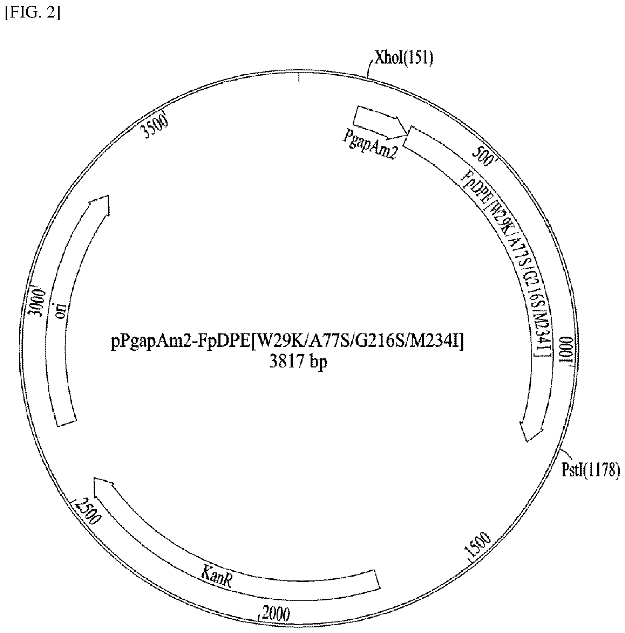

As shown in Table 6, in the case of the recombinant expression vector recovered from colonies 2, 3, 4 to 6, a mutation occurred in the allulose epimerase variant gene sequence, expecting that it will not be able to translate to make the desired allulose epimerase variant. On the other hand, in the case of the recombinant expression vector recovered from colony 1 or 5, a mutation occurred in the ribosome-binding site (RBS) sequence of the gapA promoter, but the enzyme gene sequence matched, so it was confirmed that there was a possibility of expression of the target enzyme. The variant promoter in the recombinant expression vector recovered from colony 1 was named 'gapAm1,' and the variant promoter in the recombinant expression vector recovered from colony 5 was named 'gapAm2.' The gapAm1 promoter consists of the nucleotide sequence represented by SEQ ID NO: 9, and the gapAm2 promoter consists of the nucleotide sequence represented by SEQ ID NO: 10. In addition, the recombinant expression vector recovered from colony 1 was renamed as 'pPgapAm1-FpDPE [W29K/A77S/G216S/M234I]', and the recombinant expression vector recovered from colony 5 was renamed 'pPgapAm2-FpDPE [W29K/A77S/G216S/M234I].' FIG. 1 shows a cleavage map of the recombinant expression vector pPgapAm1-FpDPE [W29K/A77S/G216S/M234I] prepared in Example of the present invention. FIG. 2 shows a cleavage map of the recombinant expression vector pPgapAm2-FpDPE [W29K/A77S/G216S/M234I] prepared in Example of the present invention.

### 4.2 Preparation of D-allulose epimerase variant expression vector having BLMA promoter

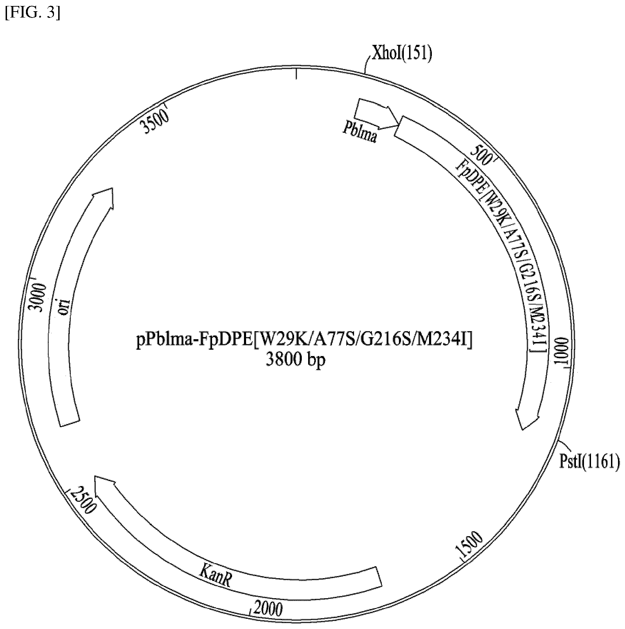

A recombinant plasmid vector containing pUC-derived replication origin that can be replicated in *E. coli* through genetic manipulation from the pTrc99A vector (Pharmacia, US), a multiple cloning site (MCS) such as restriction enzymes XhoI site and PstI site, a transcription terminator, and a kanamycin antibiotic resistance gene was constructed. Then, the polynucleotide fragment Pblma-FpDPE prepared in Example 3 was digested with restriction enzymes XhoI and PstI, and then ligated with the recombinant plasmid vector having the same restriction enzyme site to prepare D-allulose epimerase variant expression vector pPblma-FpDPE [W29K/A77S/G216S/M234I]. Thereafter, the D-allulose epimerase variant expression vector was transformed into *E. coli* DH5α by a heat shock method (See Sambrook and Russell: Molecular Cloning) to secure kanamycin-resistant colonies, six colonies were selected, then six recombinant expression vectors pPblma-FpDPE [W29K/A77S/G216S/M234I] were recovered, and their nucleotide sequences were analyzed. As a result of sequencing of six types of recombinant expression vector pPblma-FpDPE [W29K/A77S/G216S/M234I], it was confirmed that the BLMA promoter and allulose epimerase variant gene sequences were present as intended. FIG. 3 shows a cleavage map of the recombinant expression vector pPblma-FpDPE [W29K/A77S/G216S/M234I] prepared in Example of the present invention.

### Example 5: Preparation of transformants by D-allulose epimerase variant expression vector

The recombinant expression vectors pPgapAm1-FpDPE [W29K/A77S/G216S/M234I], pPgapAm2-FpDPE [W29K/A77S/G216S/M234I] and pPblma-FpDPE [W29K/A77S/G216S/M234I], respectively, prepared in Example 4 were introduced into *E.coli* W3110 by heat shock method. Thereafter, kanamycin antibiotic resistance was checked and recombinant strains transformed with the recombinant expression vector were selected. A glycerin solution was added to the prepared recombinant *E. coli* to a final concentration of 20% (v/v), and stored frozen at -70°C before culturing for enzyme expression.

### Example 6: Measurement of conversion rate of fructose to allulose by recombinant strain and comparison of promoter enzyme expression intensity

D-allulose epimerase can convert fructose to allulose, thus the intensity of enzyme expression induction of each promoter in the recombinant strain was compared by measuring the conversion rate of fructose to allulose in proportion to the enzyme expression level of the recombinant strain.

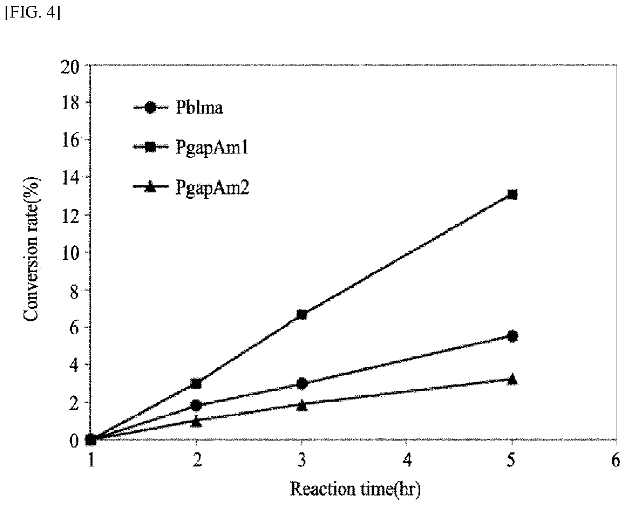

To culture the recombinant *E. coli* transformed with the recombinant expression vector, 100 ml of LB medium containing kanamycin at a final concentration of 50 µg/ml was placed in a 1 L flask, and 1 ml of the recombinant *E. coli* prepared in Example 5 was inoculated thereto. Thereafter, the flask was transferred to a shaking incubator, and the recombinant *Escherichia coli* was cultured for 14 hours while maintaining a temperature condition of 30°C. and a shaking condition of 140 rpm, and the culture medium was centrifuged to recover cells. Thereafter, the recovered cells were added at a concentration of 1 mg/ml to 50 mM PIPES buffer (pH 7.0) containing 30% (w/w) fructose and 1 mM manganese sulfate (MnSO₄) metal ions, the reaction was carried out at 62°C for a predetermined time, then the reaction was stopped by lowering the temperature of the reaction product to 4°C, and the supernatant was recovered by centrifugation at 16,600 × g and 4°C. Thereafter, the allulose concentration and the fructose concentration in the supernatant were measured using high-performance liquid chromatography (HPLC), and the conversion rate of fructose to allulose was calculated from the measured results, and then the conversion rate was used as an index of enzyme activity. FIG. 4 shows the conversion rate according to the reaction time when the conversion reaction of fructose to allulose is carried out using the recombinant *E. coli* prepared in Example of the present invention. In FIG. 4, 'PgapAm1' represents recombinant *E. coli* transformed with the recombinant expression vector pPgapAm1-FpDPE [W29K/A77S/G216S/M234I], 'PgapAm2' represents recombinant *E. coli* transformed with the recombinant expression vector pPgapAm2-FpDPE [W29K/A77S/G216S/M234I], and 'Pblma' represents recombinant *E. coli* transformed with the recombinant expression vector pPblma-FpDPE [W29K/A77S/G216S/M234I]. As shown in FIG. 4, the recombinant *Escherichia coli* introduced with the gapAm1 promoter showed a very high conversion rate of fructose to allulose, compared to the recombinant *E. coli* introduced with the gapAm2 promoter or the BLMA promoter, and from these results, it can be seen that the effect of inducing enzyme expression of the gapAm1 promoter is very strong compared to that of the gapAm2 promoter or the BLMA promoter.

### Example 7: Deposit of recombinant strains

The recombinant *Escherichia coli* transformed with the recombinant expression vector pPgapAm1-FpDPE [W29K/A77S/G216S/M234I], which has the highest conversion activity of fructose to allulose in Example 6, was named 'DS00004'. Further, on December 14, 2021, the recombinant *Escherichia coli* DS00004 was deposited as an international patent according to the Budapest Treaty at the Korean Culture Center of Microorganisms (KCCM) (Address: 3F Yurim Building, 45 Hongjenae 2ga-gil, Seodaemun-gu, Seoul, Korea), an authorized depository organization, and was given accession number KCCM13092BP. The recombinant *Escherichia coli* DS00004 (accession number: KCCM13092P) may be distributed to a third party within the procedures stipulated in the Budapest Treaty.

### [Accession Information]

### Name of depository organization: Korea Culture Center of Microorganisms (overseas)

### Accession number: KCCM13092P

### Accession date: 20211214

As described above, the present invention has been described through the above embodiments, but the present invention is not necessarily limited thereto, and various modifications may be made without departing from the scope and spirit of the present invention. Accordingly, the protection scope of the present invention should be construed to include all embodiments falling within the scope of the claims appended hereto.

## Claims

1. A promoter variant consisting of the nucleotide sequence represented by SEQ ID NO: 9.

2. A recombinant vector comprising the promoter variant of claim 1.

3. An expression vector comprising a polynucleotide encoding a target protein and the promoter variant of claim 1 operably linked thereto.

4. The expression vector of claim 3, wherein the target protein is an enzyme.

5. The expression vector of claim 4, wherein the enzyme is an allulose epimerase.

6. The expression vector of claim 5, wherein the allulose epimerase consists of an amino acid sequence represented by SEQ ID NO: 3, an amino acid sequence represented by SEQ ID NO: 5, or an amino acid sequence represented by SEQ ID NO: 7.

7. The expression vector of claim 5, wherein the polynucleotide encoding the allulose epimerase consists of a nucleotide sequence represented by SEQ ID NO: 4, a nucleotide sequence represented by SEQ ID NO: 6, or a nucleotide sequence represented by SEQ ID NO: 8.

8. A recombinant strain transformed with the expression vector of claim 3.

9. The recombinant strain of claim 8, wherein the recombinant strain is recombinant *Escherichia coli* DS00004 (accession number: KCCM13092P).

10. A method for producing allulose from fructose, the method comprising adding and reacting the recombinant strain of claim 9 to a fructose-containing solution.
